# EUROPÄISCHE PATENTSCHRIFT

(11) **EP 0 405 250 B1**
(45) Veröffentlichungstag und Bekanntmachung des Hinweises auf die Patenterteilung: **10.08.1994**
(21) Anmeldenummer: 90111319.1
(22) Anmeldetag: 15.06.1990
(51) Int. Cl.: A61B 17/39, A61B 1/12

(54) **Resektoskop**
Resectoscope
Résectoscope

(30) Priorität: 27.06.1989 DE 3921000
(43) Veröffentlichungstag der Anmeldung: 02.01.1991
(73) Patentinhaber: Richard Wolf GmbH, 75438 Knittlingen (DE)
(72) Erfinder: Bonnet, Ludwig, D-7134 Knittlingen (DE)
(74) Vertreter: Wilcken, Thomas, Dipl.-Ing.

(56) Entgegenhaltungen:
- DE-A- 2 637 747
- DE-U- 7 426 959
- GB-A- 2 002 236
- US-A- 4 068 667
- US-A- 4 132 227

## Beschreibung

Die Erfindung geht aus von einem Resektoskop nach dem Oberbegriff des Patentanspruches 1. Ein solches Resektoskop ist dem DE-U-74 26 959 beschrieben. Weitere Resektoskope sind in dem DE-U-76 26 244 und in der DE-A-26 17 556 offenbart.

Derartige Resektoskope besitzen einen Außenschaft und einen am distalen Ende ein hohlzylindrisches Isolierelement aufweisenden Innenschaft für die Durchführung einer isolierten, durch einen Transporteur verschiebbaren Stromzuführung einer distalen Resektionsschlinge und mit einer Optik, wobei der freibleibende Raum des Innenschaftes zum Zuführen einer Spülflüssigkeit und der Raum zwischen Außen- und Innenschaft zum Abführen der Spülflüssigkeit über distale Öffnungen des Außenschaftes dient sowie in einem proximalen, absperrbaren Anschlußstutzen endet.

Diese bekannten Resektoskope besitzen, um einen ausreichenden Spülflüssigkeitsumlauf zu erreichen, einen großen Schaftquerschnitt, insbesondere am distalen Ende, was zu einem erschwerten Einführen in die Harnröhre oder sogar zu Verletzungen der Harnröhrenschleimhaut führt.

Die Aufgabe der Erfindung besteht darin, bei Resektoskopen der eingangs erwähnten Art ein stabiles Isolierelement am distalen Ende des Innenschaftes vorzusehen, welches einen Stromübergang von der Resektionsschlinge zum Außenschaft verhindert, dabei aber eine ausreichende Spülmittelabfuhr aus der Blase bei einem möglichst geringen Querschnitt des Außenschaftes gewährleistet.

Diese Aufgabe wird durch die kennzeichenmerkmale des Anspruches 1 gelöst.

Durch diese Lösung ist es möglich, eine einwandfreie Isolierung der Resektionsschlinge gegen den Schaft und gegen den Benutzer des Resektoskopes bei einer für das Einführen in die Harnröhre stabilen Ausführung des distalen Instrumentenendes zu erreichen und trotz der stabilen Ausführung des Isolierelementes einen ausreichend großen Raum zwischen dem Außen- und Innenschaft für das Abführen der Flüssigkeit aus der Blase zu gewährleisten. Durch die Umfangsausnehmung des Isolierelementes ist dieser Raum vorhanden und gleichzeitig dadurch der Querschnitt des Resektoskopes am distalen Ende verhältnismäßig klein gehalten.

Die Erfindung ist nachstehend anhand der Zeichnung beschrieben. Es zeigen:

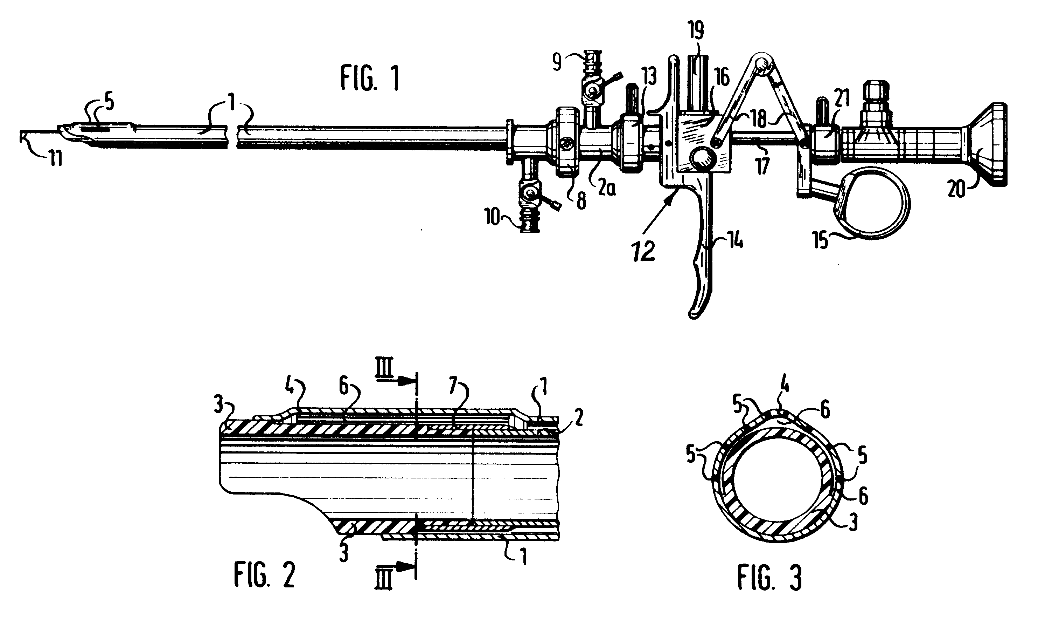

- Figur 1: das Endoskop nach der Erfindung in Seitenansicht,
- Figur 2: einen Längsschnitt durch das distale Ende des Resektoskopes nach Figur 1 in Vergrößerung jedoch ohne Darstellung der Resektionsschlinge,
- Figur 3: einen Schnitt nach der Linie III-III der Figur 1.

Das Resektoskop nach der Erfindung besteht aus einem Außenschaft 1 und einem Innenschaft 2. Der Innenschaft endet distal in einem über das distale Ende des Außenschaftes hinausgehenden Isolierelement 3. Der Außenschaft ist am distalen Ende auf der längsgerichteten Oberseite über eine bestimmte Länge mit einer Erweiterung 4 versehen, die mehrere Längsschlitze 5 aufweist. Das Isolierelement 3 ist etwa über die Länge der Erweiterung 4 auf seiner Außenfläche bzw. auf seinem Außenmantel mit einer Ausnehmung 6 versehen, die sich teilringzylindrisch über den größeren Teil des Umfanges erstreckt. Das Isolierelement 3 stößt proximal stumpf an das distale Ende des Innenschaftes 2 an und ist mit dem Innenschaft durch einen Zylinderring 7 unlösbar verbunden.

Der Innenschaft 2 endet proximal in einer im proximalen Ende des Außenschaftes 1 durch einen Spannring 8 abgedichteten Erweiterung 2a, die mit einem Absperrhahn 9 für das Zuführen einer Spülflüssigkeit in den Innenraum des Innenschaftes 2 versehen ist. Der distale Raum zwischen dem Außenschaft 1 und dem Isolierelement 3 ist beim Gebrauch über die Schlitze 5 mit der zu bespülenden Blase verbunden und endet proximal in einem Absperrhahn 10 für den Abzug der der Blase zugeführten Spülflüssigkeit.

Zur Resektion wird in den Innenschaft ein die Schneidschlinge 11 in axialer Richtung betätigender Elektrodentransporteur 12 eingeführt, der durch eine Konuskupplung mittels eines Spannringes 13 lösbar an den Innenschaft 2 angeschlossen ist. Die Schneidbewegung der Schneidschlinge 11 erfolgt dadurch, daß der Handgriffteil 14 gegen den ortsfesten Handgriffteil 15 bewegt wird, wodurch der Schloßkörper 16 auf der Führung 17 gegen die Wirkung der Schenkelfelder 18 zurückgezogen wird. Die Schneidschlinge 11 ist im Schloßkörper 16 austauschbar festgelegt und über den HF-Anschluß 19 an eine Stromquelle angeschlossen.

Die durchzuführende Resektion ist durch eine durch den Innenschaft verlaufende Optik mit Okular 20 zu beobachten, die wieder durch eine Kegelkupplung mit Spannring 21 lösbar festgelegt ist.

Durch das Isolierelement 3 ist eine Isolation zwischen den beiden Schäften 1 und 2 erreicht und es ist verhindert, daß ein Stromübergang von der Schneidschlinge auf die Schäfte erfolgt.

## Patentansprüche

1. Resektoskop mit einem Außenschaft (1) und einem am distalen Ende ein hohlzylindrisches Isolierelement (3) aufweisenden Innenschaft (2) für die Durchführung einer isolierten, durch einen Transporteur (12) verschiebbaren Stromzuführung einer distalen Resektionsschlinge (11) und mit einer Optik, wobei der freibleibende Raum des Innenschaftes (2) zum Zuführen einer Spülflüssigkeit und der Raum zwischen Außen- und Innenschaft zum Abführen der Spülflüssigkeit über distale Öffnungen (5) des Außenschaftes (1) dient sowie in einem proximalen, absperrbaren Anschlußstutzen (10) endet, dadurch gekennzeichnet, daß das distale Isolierelement (3) des Innenschaftes (2) über eine vom distalen Außenschaftende (4) übergriffene Länge am Außenmantel mit einer sich teilringzylindrisch über den größeren Teil des Umfanges erstreckenden Ausnehmung (6) versehen ist und die distalen Öffnungen (5) als Schlitze ausgebildet sind.

2. Resektoskop nach Anspruch 1, dadurch gekennzeichnet, daß das Isolierelement (3) am proximalen, im Außendurchmesser auf den Außendurchmesser des Innenschaftes (2) verringerten Ende mittels eines Zylinderringes (7) mit dem distalen Ende des Innenschaftes (2) unlösbar verbunden ist.

3. Resektoskop nach Anspruch 1, dadurch gekennzeichnet, daß das Isolierelement (3) zur Aufnahme des Innenschaftes (2) eine zylindrische Ausnehmung aufweist.

## Claims

1. Resectoscope having an outer shaft (1) and an inner shaft (2) having a hollow cylindrical insulating element (3) at the distal end for guiding an insulated current supply, which can be displaced by means of a transporter (12), to a distal resection loop (11) and having an optical system, the space remaining free in the inner shaft (2) serving to supply a rinsing fluid and the space between outer and inner shaft serving to remove the rinsing fluid via distal openings (5) in the outer shaft (1), and terminating in a proximal, blockable connection piece (10), characterised in that the distal insulating element (3) of the inner shaft (2) is provided with a cylindrical recess (6) extending like a cylindrical graduated ring over the greater part of the periphery on the outer wall over a length overlapped by the distal end (4) of the outer shaft, and the distal openings (5) are designed as slits.

2. Resectoscope according to claim 1, characterised in that the insulating element (3) is non-releasably connected to the distal end of the inner shaft (2) by means of a cylindrical ring (7) at the proximal end with the outer diameter reduced to the outer diameter of the inner shaft (2).

3. Resectoscope according to claim 1, characterised in that the insulating element (3) has a cylindrical recess to receive the inner shaft (2).

## Revendications

1. Résectoscope pourvu d'une tige externe (1) et d'une tige interne (2) présentant à l'extrémité distale un élément isolant creux cylindrique (3) pour effectuer un acheminement isolé de courant déplaçable par un transporteur (12) dans une boucle de résection distale (11) et comportant une optique, l'espace restant de la tige interne (2) servant à l'acheminement d'un liquide de lavage et l'espace entre la tige externe et la tige interne servant à l'évacuation du liquide de lavage via les ouvertures distales (5) de la tige externe (1) et se terminant par un raccord proximal blocable (10), caractérisé en ce que l'élément isolant distal (3) de la tige interne (2) sur une longueur de l'enveloppe externe sur laquelle empiète l'extrémité distale (4) de la tige externe, d'un évidement (6) s'étendant sous une forme cylindrique partiellement annulaire sur la plus grande partie de la périphérie et les ouvertures distales (5) se présentent sous la forme de fentes.

2. Résectoscope selon la revendication 1, caractérisé en ce que l'élément isolant (3) est relié de manière inamovible à l'extrémité distale de la tige interne (2) par une bague cylindrique (7) sur l'extrémité proximale dont le diamètre externe est réduit au diamètre externe de la tige interne (2).

3. Résectoscope selon la revendication 1, caractérisé en ce que l'élément isolant (3) présente un évidement cylindrique pour recevoir la tige interne (2).
